# EUROPÄISCHE PATENTANMELDUNG

(11) **EP 1 544 621 A1**
(43) Veröffentlichungstag der Anmeldung: **22.06.2005**
(21) Anmeldenummer: 04027716.2
(22) Anmeldetag: 23.11.2004
(51) Int. Cl.: G01N 33/86, G01N 33/96

(54) **Kontrollplasmen für Thrombinaktivitätstests**

(30) Priorität: 19.12.2003 DE 10360628
(71) Anmelder: Dade Behring Marburg GmbH, 35001 Marburg (DE)
(72) Erfinder: Henckel, Thilo, Dr., 35083 Wetter (DE)

(57) **Zusammenfassung**

Delipidisierte Plasmazubereitungen, welche definiert eingestellte Prothrombinkonzentrationen aufweisen, die niedriger oder höher sind als die von Normalhumanplasma, eignen sich zur Kalibrierung, Standardisierung und Kontrolle von Thrombinaktivitäts- und Thrombingenerierungstests wie z. B. ETP-Tests. Die Plasmazubereitungen werden unter Verwendung eines Prothrombin-Mangelplasmas hergestellt, das z. B. durch Immunadsorption aus Normalplasma erhalten wird, und können zusätzlich Puffersubstanzen, Proteinaseinhibitoren, Antikoagulantien, Stabilisatoren und/oder andere bei der Plasmaaufbereitung gebräuchliche Hilfs- und Zusatzstoffe enthalten.

## Beschreibung

Die vorliegende Erfindung betrifft Referenzplasmaprodukte, insbesondere Referenzplasmaprodukte definierter Prothrombinkonzentration und -Aktivität, die insbesondere zur Kontrolle und Kalibration von Thrombinaktivitäts- und Thrombingenerierungstests eingesetzt werden können. Bevorzugt werden sie bei Verfahren zur Bestimmung des endogenen Thrombinpotentials (ETP-Tests) verwendet.

Bei einem ETP-Test im Sinne der vorliegenden Erfindung handelt es sich um einen globalen Gerinnungstest, mit dem die Bildung und Inhibition von Thrombin bestimmt werden. Unter dem endogenen Thrombinpotential (ETP) versteht man das einer Probe innewohnende (endogene), im Falle von Plasmaproben plasmaeigene Vermögen (Potential) zur Bildung und Inhibition von enzymatisch aktivem, freiem Thrombin.
Ein Parameter, der zur Quantifizierung des endogenen Thrombinpotentials bevorzugt bestimmt wird und der in der Literatur auch als "endogenes Thrombinpotential" bezeichnet wird, ist das Zeit-/Konzentrationsintegral oder die Fläche unter der Thrombinbildungskurve (siehe EP0420 332 B1). Dieser Parameter ist ein Maß für die Menge und Aktivität von endogenem Thrombin, welches seit einer Zeit t = 0 in einer Probe von gerinnendem Blut oder Plasma anwesend war.
Die Bestimmung des endogenen Thrombinpotentials ist eine wichtige Voraussetzung, um z. B. eine wirksame Behandlung an Mensch oder Tier mit Antithrombotika vorzunehmen und diese über den Behandlungszeitraum zuverlässig zu kontrollieren. Ein ETP-Test kann universell für alle Antithrombotika verwendet werden und ist damit anderen aus dem Stand der Technik bekannten Gerinnungstests, wie z. B. Bestimmung der Gerinnungszeit mit Hilfe von Gewebethromboplastin (Prothrombinzeit = PT) oder Bestimmung der Gerinnungszeit mit Hilfe von Kontaktaktivatoren und Phospholipiden (aktivierte teilweise Thrombinplastinzeit = APTT), überlegen. So ist das PT-Verfahren gegenüber oraler Antigerinnung empfindlich, aber unempfindlich gegenüber Heparin. Während das APTT-Verfahren gegenüber Heparin und oraler Antigerinnung, aber nicht oder praktisch nicht gegenüber Heparinen mit niedrigem Molekulargewicht oder gegenüber' Dermatansulfat empfindlich ist. Weiterhin werden mit dem ETP thrombogene Risikofaktoren, wie z. B. die Erhöhung des Thromboserisikos durch orale Kontrazeption (Einnahme der "Pille"), Rauchen oder Schwangerschaft erfasst, welche in herkömmlichen Globaltests nicht erfasst werden.

Die Messungen werden bei ETP-Testen z. B. mit einem Photometer (z. B. Messung der optischen Dichte) oder einem Fluorimeter durchgeführt. Dazu können auch geeignete automatische Gerinnungsanalysatoren verwendet werden. Die mit den jeweiligen Geräten erhaltenen Messwerte sind zunächst absolute Werte ohne Bezugssytem und werden daher auch als Rohwerte bezeichnet. Sie sind geräte-, reagenz- und testspezifisch und daher nicht direkt miteinander vergleichbar und somit auch nicht direkt einem bestimmten physiologischen Zustand zuzuordnen. Eine Standardisierung eines ETP-Tests ist bisher noch nicht erfolgt, auch bedingt durch die Nichtexistenz geeigneter Kalibrierreagenzien.

Die Bedeutung, auch im Vergleich zum Stand der Technik, die Durchführung und Auswertung des ETP-Tests sind z. B. in der EP 0 420 332 B1, EP 0 802 986 B1 und der dort zitierten Literatur beschrieben.

Um die absoluten Messergebnisse von enzymatischen Aktivitätstests in ein Bezugssystem einordnen zu können, bedarf es Kalibrations- und/oder Kontrollsubstanzen mit bekannten, definierten Werten, also einer bekannten Enzymkonzentration und/oder enzymatischen Aktivität. Erst dadurch werden Messergebnisse vergleichbar und standardisierbar. Kontrollsubstanzen sind weiterhin notwendig, um dem Anwender die Richtigkeit des Messsystems und der Messergebnisse anzuzeigen. Dies ist insbesondere von Bedeutung, um eine Kontrolle der Richtigkeit und Zuverlässigkeit des Testablaufs im pathologischen Probenbereich, z. B. bei einer Behandlung von Patienten mit Antithrombotika, zu gewährleisten.

Für die aus dem Stand der Technik bekannten PT- und APTT-Verfahren sind solche Kontrollsubstanzen und auch Kontrollplasmen bekannt. So wird in der WO 01/07921 A2 eine Plasmamischung beschrieben, die neben dem Hauptbestandteil Primatenplasma, Nicht-Primatenplasma und Stabilisatoren wie z. B. Puffersubstanzen und Fibrinolyseinhibitoren enthält. In der WO 95/12127 werden lyophilisierte Plasmaproben zur Kalibrierung des PT-Verfahrens beansprucht, die spezielle IRP-Werte (International Reference Preparation) für Thromboplastin aufweisen. In der WO 00/02054 werden Plasmen beschrieben, die ein abnormales Plasma, d. h. eine Mischung aus Primaten- und Nicht-Primatenplasma, und ein Antikoagulanz umfassen. Schließlich bezieht sich die EP 0 482 088 B1 auf stabile Kontrollplasmen, die neben Nicht-Primatenplasma wirksame Gehalte an Puffersubstanzen, Proteaseinhibitoren und stabilisierenden Kohlenhydraten enthalten.

Wie oben bereits ausgeführt existieren für Thrombinaktivitäts- und Thrombingenerierungstests, wie z. B. ETP-Tests, bisher keine solchen Kontroll-, Referenz- oder Kalibrationssubstanzen: Der Anwender muss sich damit behelfen, dass durch Messung eines Normalplasmas oder Normalplasmapools ein Normalwert festgelegt wird. Zu diesem Normalwert werden dann erhaltene. Probenwerte durch ein lineares Dreisatzverfahren in Relation gesetzt und z. B. in % der Norm, Units oder nmol x min angegeben (vgl. z. B. Beschreibung des ETP-Tests in EP 0 420 332 B1).

Aufgabe der vorliegenden Erfindung war daher die Bereitstellung von Referenzoder Kontroll- bzw. Kalibriersubstanzen, die insbesondere zur Standardisierung, Kalibration und Richtigkeitskontrolle von Thrombinaktivitäts- und Thrombingenerierungstests, bevorzugt eines ETP-Tests, verwendet werden können.

Diese Aufgabe wird durch die in den Ansprüchen beschriebenen Gegenstände und Verfahren, insbesondere durch die erfindungsgemäßen Plasmaprodukte, gelöst.

Erfindungsgemäße Plasmaprodukte enthalten ein oder mehrere humane oder tierische Prothrombin-Mangelplasmen (Faktor II-Mangelplasmen) oder Mischungen davon. Ferner können sie auch humanes oder tierisches Normalplasma enthalten. Die Prothrombin-Mangelplasmen, die Normalplasmen und/oder das erfindungsgemäße Plasmaprodukt können bevorzugterweise delipidisiert sein. Delipidisierte Plasmen können lyophilisiert werden und sind daher sehr lange ohne Aktivitätsverlust haltbar.

Bevorzugterweise können die erfindungsgemäßen Plasmaprodukte defibriniert werden. Zur Vermeidung von Fibringerinnseln während der kontinuierlichen Messung der Thrombinbildung und -inhibition, vorzugsweise in chromogenen Thrombingenerierungstesten, können entweder Inhibitoren der Fibrinaggregation bzw. -polymerisation, sogenannte Clot-Inhibitoren, dem Reagenzansatz zugegeben werden oder zuvor das Fibrinogen aus dem Plasma entfernt werden. Geeignete Clot-Inhibitoren sind z. B. Pefabloc®FG H-Gly-Pro-Arg-Pro-OH-AcOH (Pentapharm Ltd, Schweiz) oder Peptidamide, wie sie in EP 0 456 152-B1 beschrieben sind.
Die Defibrinierung des Plasmas kann durch verschiedene, dem Experten bekannte Methoden bewerkstelligt werden, z. B. durch die Zugabe von Schlangengiften wie beispielsweise Batroxobin, durch Hitze, durch Ausfällung oder Immunaffinitätschromatographie.
Die Defibrinierung der erfindungsgemäßen Plasmaprodukte macht eine generelle Anwendung der Kalibrierplasmen sowohl in Tests mit Clot-Inhibitor als auch ohne Clot-Inhibitor möglich.

Bei einer bevorzugten Ausführungsform werden Plasmaprodukte humanen Ursprungs mit unterschiedlicher und definierter Prothrombinkonzentration (Prothrombin = Faktor II = FII) bzw. Thrombinaktivität (Thrombin = Faktor IIa = Flla) hergestellt. Diese unterschiedliche Thrombinaktivität des Plasmas wird durch verschiedene, definierte Prothrombinkonzentrationen in dem Plasma erzeugt bzw. definiert eingestellt. Dazu kann die Prothrombinkonzentration und damit die Thrombinaktivität des Plasmas von niedrigen bis zu sehr hohen Werten eingestellt werden, d. h. die Prothrombinkonzentration bzw. Thrombinaktivität des erfindungsgemäßen Plasmaproduktes kann sowohl unterhalb als auch oberhalb der entsprechenden Werte eines Normalplasmas bzw. Normalplasmapools liegen. Durch eine quantitative Bestimmung des endogenen Thrombinpotentials dieser definierten Plasmaprodukte werden Messwerte (ETP-Rohwerte) erzeugt, die zur Prothrombinkonzentration oder Thrombinaktivität des Plasmas in Relation gesetzt werden. Es entsteht so eine Kalibrationskurve, an der unbekannte Proben bzw. deren Probenmesswerte unter Zuhilfenahme eines geeigneten mathematischen Algorithmus bestimmt werden können.

Genauso können einzelne Plasmen mit niedrigen, normalen und über dem Normalwert liegenden. Prothrombinkonzentration bzw. Thrombinaktivitäten als Kontrollplasmen zur Überprüfung der Richtigkeit des Messsystems dienen.

Aus Vereinfachungsgründen werden die erfindungsgemäßen Plasmaprodukte zusammenfassend und unabhängig von ihrer möglichen Verwendung als Kontrollplasmen bezeichnet.

Somit können die erfindungsgemäßen Kontrollplasmen z. B. zur Standardisierung und Kalibration von enzymatischen Aktivitätstests, welche die Thrombinaktivität bestimmen, verwendet werden. Die Quantifizierung der Prothrombinkonzentration und/oder Thrombinaktivität einer unbekannten Probe, welche unter oder über der normalen Prothrombinkonzentration und/oder Thrombinaktivität liegen kann, wird dadurch ermöglicht.

Damit ergeben sich insbesondere die folgenden Anwendungsgebiete für die genannten Kontrollplasmen: Kalibrierung, Standardisierung und/oder Kontrolle von Thrombingenerierungstesten wie z. B. ETP-Testen, von Thrombinaktivitäts-tests, Prothrombintests oder von enzymatischen Tests, bei denen die Thrombin-aktivität oder -konzentration über die Aktivierung von Prothrombin bestimmt wird. Die Kontrollplasmen eignen sich ferner zur Simulation pathologischer Gerinnungszustände. Hyper- oder auch hypokoagulatorische Zustände, verursacht durch veränderte Prothrombin- oder Thrombinbildung, können dargestellt werden.

Kalibrations- oder Referenzkurven können durch Verwendung eines Kalibriersets, bestehend aus mindestens 2 erfindungsgemäßen Kontrollplasmen unterschiedlicher FII-Konzentration, oder durch Verdünnung eines hochkonzentrierten Kontrollplasmas mit einem geeignetem Verdünnungsmedium erstellt werden:
1. Verwendung eines Kalibrierplasmasets bestehend aus mehreren Kontrollplasmen unterschiedlicher FII-Konzentration:
   Die endogenen Thrombinpotentiale einer bestimmten Anzahl von Kontrollplasmen mit unterschiedlicher FII-Konzentration werden gemessen und den enstprechenden Prothrombinkonzentrationen der jeweiligen Kontrollplasmen zugeordnet. Es besteht ein direkt proportionaler, linearer Zusammenhang zwischen den bestimmten Messwerten und der Prothrombinkonzentration des Kontrollplasmas. Mit einem geeignetem Algorithmus kann die Prothrombinkonzentration oder auch die Prothrombinaktivität unbekannter Proben berechnet werden.
2. Verwendung eines Kalibrierplasmasets bestehend aus einem Kontrollplasma hoher FII-Konzentration und einem Verdünnungsmedium:
   Ein Kontrollplasma mit hoher Prothrombinkonzentration wird bevorzugterweise mit FII-Mangelplasma seriell verdünnt. Idealerweise sollte die Prothrombinkonzentration des zu verdünnenden Kontrollplasmas die maximal zu erwartenden Probenwerte übertreffen. Eine etwa dreifache Normalkonzentration sollte ausreichen, um auch hyperkoagulabile Proben unterhalb des maximalen Wertes der Referenzkurve bestimmen zu können. Die endogenen Thrombinpotentiale werden quantitativ bestimmt und den entsprechenden Konzentrationen der einzelnen Verdünnungsstufen zugeordnet. Es besteht ein direkt proportionaler, linearer Zusammenhang zwischen den bestimmten Messwerten und der Prothrombinkonzentration der verschiedenen Verdünnungsstufen. Mit einem geeignetem Algorithmus kann die Prothrombinkonzentration oder auch die Prothrombinaktivität unbekannter Proben berechnet werden.

Im Folgenden wird das Herstellungsverfahren eines erfindungsgemäßen Kontrollplasmas näher beschrieben:
a) Herstellung von Kontrollplasmen mit subnormaler bis normaler Thrombinaktivität:
   Durch Auswahl offensichtlich gesunder Blutspender (Mensch oder Tier) werden ein Normalplasmapool und/oder einzelne Normalplasmen hergestellt. Der Normalplasmapool bzw. das Normalplasma wird durch Adsorption, z.B. unter Verwendung von Prothrombin-spezifischen monoklonalen und/oder polyklonalen Antikörpern, von Prothrombin befreit oder weitgehend befreit, wodurch ein sogenanntes FII-Mangelplasma erhalten wird. Dieses FII-Mangelplasma wird durch die folgenden Verfahren A) oder B) auf bestimmte FII-Konzentrationen und Aktivitäten eingestellt.
      A) Zugabe definierter Anteile eines Normalplasmas oder Normalplasmapools zu FII-Mangelplasma, wodurch FII-Konzentrationen bis annähernd der FII-Konzentration gesunder Blutspender erreicht werden können.
      B) Zugabe von reinem Prothrombin oder Prothrombin-Konzentrat zu FII-Mangelplasma.
b) Herstellung von Kontrollplasmen mit Thrombinaktivitäten über normalen Werten:
   Ein Normalplasmapool, ein Normalplasma oder ein FII-Mangelplasma werden mit gereinigtem FII oder einem Prothrombin-Konzentrat auf supernormale FII-Konzentration eingestellt, wodurch bevorzugterweise FII-Konzentrationen von über 1,4 µM bzw. eine FIIa-Aktivität von über 1 U/ml erreicht werden. 1 U ist die Aktivität von FIIa, die benötigt wird, um eine normale Gerinnungszeit (Thromboplastinzeit oder Quickwert) von 70-130 % der Norm, zu erreichen. Eine FII-Konzentration von 90 µg pro mL Plasma, bzw. 1,4 µM, bzw. 1 Unit FIIa-Aktivität per ml Plasma entsprechen einem FII-Normalwert.

Humanes, tierisches oder gentechnologisch hergestelltes Prothrombin kann für die erfindungsgemäßen Kontrollplasmen verwendet werden. Besonders bevorzugt ist rekombinant hergestelltes, humanes Prothrombin.

Die erfindungsgemäßen Kontrollplasmen können frisch verwendet werden. Wenn eine längere Lagerung über mehrere Wochen erwünscht ist, sollten die Kontrollplasmen tiefgefroren oder lyophilisiert gelagert werden.

Durch Zugabe von geeigneten Stabilisatoren (z. B. Hepes, Tris; vgl. WO 01/07921 A2, Seite 8) können die Kontrollplasmen über lange Zeit haltbar gemacht werden. Vor der Lyophilisation oder einer Lagerung im gefrorenen Zustand sollten die - Kontrollplasmen bevorzugt delipidisiert werden. Dies hat den Vorteil, dass solche Kontrollplasmen in chromogenen Nachweisverfahren, z. B. einem chromogenen ETP-Test, eingesetzt werden können.

Eine Delipidisierung der erfindungsgemäßen Plasmaprodukte (z. B. durch Adsorption an lipophile Substanzen oder Aufschwämmung durch Zentrifugation oder Klärung durch Lösen in nicht wasserlöslichen organischen Substanzen) ist vorteilhaft, um ein Ausfallen bzw. eine Trübung durch Lipidkolloide nach Rekonstitution der lyophilisierten oder aufgetauten Produkte zu verhindern. Durch solche Lipidkolloide kann z. B. die photometrische Messung der Thrombinbildungskinetik empfindlich gestört werden (vgl. z. B: EP 0 420 332-B1).

Besonders durch Delipidisierung und anschließende Lyophilisation wird es möglich, Kontrollplasmen definierter. Prothrombin-Konzentration und damit definierter Thrombin-Aktivität herzustellen, die eine sehr lange Haltbarkeit, auch über einen Zeitraum von 12 Monaten hinaus, aufweisen.

Es ist auch möglich auf den Delipidisierungsschritt zu verzichten. Allerdings sollten dann nach Rekonstitution entstandene Lipidkolloide und denaturiertes Protein aus den Produkten entfernt werden, z. B. durch Zentrifugation, Adsorption oder Filtration.

Die erfindungsgemäßen Kontrollplasmen können Antikoagulantien wie z. B. Natriumcitrat oder EDTA; Puffersubstanzen wie z. B. Hepes oder Tris; Proteaseinhibitoren wie z. B. Trasylol® (Aprotinin); Stabilisatoren der Koagulationsfaktoren wie z. B. Zucker oder Zuckeralkohole und/oder andere in der Plasmaaufbereitung gebräuchliche Hilfs- und Zusatzstoffe enthalten. Weitere Beispiele für die oben genannten Substanzklassen sind dem Fachmann bekannt und z. B. in der WO 01/07921 A2 ( hier insbesondere auf den Seiten 8 und 9) beschrieben, auf die hier ausdrücklich Bezug genommen wird.

Nachfolgend wird die Herstellung der erfindungsgemäßen Kontrollplasmen anhand von Beispielen detailliert beschrieben, ohne damit die Erfindung auf den Umfang der Beispiele beschränken zu wollen. Die beschriebenen Herstellungsverfahren und angegebenen Verwendungen der erfindungsgemäßen Kontrollplasmen sind ebenfalls Gegenstand der vorliegenden Erfindung.

### Beispiele:

### Beispiel 1: Herstellung eines stabilisierten Plasmapools

Es wird Blut von offensichtlich gesunden Spendern entnommen. Das Blut wird mit einer Natriumcitratkonzentration von 0,105 mol/l antikoaguliert. Zur Plasmagewinnung werden die Proben 20 ± 2 min bei 1500 x g und bei + 10 °C ungebremst zentrifugiert. Die Zentrifugation sollte innerhalb von 4 Stunden nach Blutentnahme erfolgt sein. Der Plasmaanteil jeder Einzelspende wird zu einem Pool gemischt. Dieser Pool wird durch Zugabe einer 40 %igen HEPES (N-[2-Hydroxyethyl] piperazin-N'-[2-ethansulfonsäure])-Lösung (10 ml/l Plasma) und Zugabe von Trasylol® (10 000 KIE/ml; 2 ml/l Plasma) stabilisiert und anschließend filtriert. Der pH-Wert des Normalplasmapools sollte im Normalbereich liegen, bevorzugt pH 7,3 ±0,2.

### Beispiel 2: Herstellung Faktor II-Mangelplasma

Ein Aliquot des gemäß Beispiel 1 hergestellten Normalplasmapools wird durch eine Chromatographiesäule gepumpt, die an CNBr-aktivierte Sepharose gebundene, FII-spezifische monoklonale Antikörper (Dade Behring Marburg GmbH, Deutschland) enthält. Die Fraktionen des Durchlaufs mit einer FII-Aktivität < 1 % der Norm werden aufgefangen. Eventueller Mangel an FVIII (< 70% der Norm) wird durch Zugabe von FVIII-Konzentrat (z. B. Häemate® , Aventis Behring GmbH, Deutschland) ausgeglichen. Dem Ansatz wird unter langsamem Rühren, so dass Schaumbildung vermieden wird, zur Stabilisierung D(-)-Mannit (20 g/l Plasma) zugegeben.

### Beispiel 3: Delipidisierung von Plasma

Das zu delipidisierende Plasma wird über kovalent an Sepharose Cl 2 B gekoppeltes Triton® X-1 00 gegeben. Zur Herstellung von Sepharose Cl 2 B gekoppeltem Triton® X-100, wird mittels katalytischer Wirkung von Bortrifluoridethyletherat der Glycidylether von Triton® X-100 (Roche Applied Science, Deutschland) an Sepharose Cl 2B (Pharmacia Biotech, Schweden) kovalent gebunden. Triton® X-100-Sepharose bindet Lipoproteine, während andere Proteine das Adsorbens ungebunden passieren, so dass ein klares, stabilisiertes Plasma gewonnen wird, das z. B. für die Herstellung von ETP-Referenzplasma geeignet ist.

Die Plasmaauftragsmenge steht in Abhängigkeit der Bindungskapazität der Triton® X-100 Sepharose. Sie sollte 80 % der theoretischen Bindungskapazität nicht überschreiten.

Die Triton® X-100 Sepharose-Säule wird mit 10 mM Natriumcitrat-Puffer plus 0,9 % Natriumchlorid, pH 7,4, äquilibriert. Der Plasmapool wird bei maximalem Fluss aufgetragen. Nach dem vollständigen Einlaufen des Plasmapools spült man mit ≥ 1 Gelbettvolumen Natriumcitrat nach, um alle ungebundenen Proteine zu eluieren. Nachdem ≤ 0,9 Gelbettvolumen (Plasma + Elutionspuffer) eingelaufen ist, werden Fraktionen gesammelt. Alle Fraktionen der Flankenbereiche mit einer UV-Absorption (280 nm / 1 cm Schichtdicke) von > 0,7 werden durch Schwenken unter Vermeidung von Schaumbildung gemischt.

### Beispiel 4: Defibrinierung von Plasma

Die hier verwendete Methode beinhaltet die Defibrinierung unter Verwendung von Schlangengift von *Bothrops atrox* (Batroxobin, Dade Behring Marburg GmbH, Deutschland). Plasma wird mit einer geeigneten Konzentration (z. B. 1:20 bis 1:50) Batroxobin Reagenz 30 min bei Raumtemperatur inkubiert. Anschließend wird das Plasma zweimal zentrifugiert (800 x g, 15 min) und der Überstand abgehoben. Der Überstand wird abschließend über Gaze filtriert.

### Beispiel 5: Herstellung von erfindungsgemäßen Kontrollplasmen (Zugabe von FII-Konzentrat zu FII-Mangelplasma)

Durch Zugabe von Prothrombin zu Prothrombin-freiem Plasma (FII-Mangelplasma) können beliebige Prothrombinkonzentrationen und -Aktivitäten hergestellt werden. Es können mit diesem Verfahren auch Plasmaprodukte hergestellt werden, die den FII-Normalwert übersteigen.

Beispielhaft wurden 6 verschiedene Kalibrierplasmen Level 1 bis 6 aus delipidisiertem FII-Mangelplasma (s. Beispiel 2) und Prothrombin-Konzentrat (Prothrombin-Lösung [Cat# 20-267, Fa. Milan, CH-1634 La Roche] in 50 mM Tris-HCl) mit den unten genannten Prothrombinkonzentrationen hergestellt:

Einstellung der Prothrombinkonzentration in FII-Mangelplasma:
- Level 1:: 0 µg Prothrombin / mL
- Level 2:: 18 µg Prothrombin / mL
- Level 3: 54 µg Prothrombin / mL
- Level 4:: 90 µg Prothrombin / mL
- Level 5:: 126 µg Prothrombin / mL
- Level 6:: 180 µg Prothrombin / mL

Die entsprechenden Mischungsverhältnisse FII-Mangelplasma und Prothrombin-Konzentrat richten sich nach der Ausgangskonzentration der jeweiligen Prothrombin-Charge und müssen entsprechend berechnet werden. Der Ansatz wird vorsichtig ohne Schaumbildung bis zur homogenen Durchmischung gerührt. Zur Lagerung werden die Kontrollplasmen lyophilisiert oder tiefgefroren (- 70 °C).

### Beispiel 6: Herstellung von erfindungsgemäßen Kontrollplasmen (Zugabe von FII-Mangelplasma zu Normalplasma)

Durch Zugabe von FII-Mangelplasma (s. Beispiel 2) zu Normalplasma (s. Beispiel 1) können Prothrombinkonzentrationen und Aktivitäten hergestellt werden, die den FII-Normalwert (ca. 1,4 µM FII) nicht übersteigen.

Die entsprechenden Mischungsverhältnisse FII-Mangelplasma und Normalplasma (NP) werden entsprechend berechnet. Hierbei wird die Aktivität eines humanen Normalplasmas mit 1 U/ml definiert. 1 U ist die Aktivität von Flla, die benötigt wird um eine normale Gerinnungszeit (Thromboplastinzeit oder Quickwert) von 70-130 % der Norm zu erreichen. Daraus ergeben sich folgende Aktivitäten:

### Beispiel:

- Level 1:: 8 Volumenteile FII-Mangelplasma + 2 Volumenteile NP → 0,2U/ml
- Level 2:: 6 Volumenteile FII-Mangelplasma + 4 Volumenteile NP → 0,4U/ml
- Level 3:: 4 Volumenteile FII-Mangelplasma + 6 Volumenteile NP → 0,6 U/ml
- Level 4:: 2 Volumenteile FII-Mangelplasma + 8 Volumenteile NP → 0,8 U/ml
- Level 5:: NP → 1,0 U/ml

Der Ansatz wird vorsichtig ohne Schaumbildung bis zur homogenen Durchmischung gerührt. Zur Lagerung werden die Plasmen lyophilisiert oder tiefgefroren (-70°C).

### Beispiel 7: Bestimmung der FII-Aktivität

Die FII-Aktivität bzw. die Thrombin-Aktivität kann gemäß Herstellerangaben (Gerinnungsfaktor-II-Mangelplasma, Bestell-Nr. OSGR, Dade Behring Marburg GmbH, Deutschland) über die Verlängerung der Prothrombinzeit (PT) einer Probe bestimmt werden. Zur Einzelfaktorbestimmung wird die PT einer Mischung des FII-Mangelplasma mit einer zu bestimmenden Probe gemessen. Die Aktivität des FII in % der Norm wird über eine Bezugskurve ermittelt, die mit Verdünnungen von Normalplasmapool oder Standardhumanplasma (Standard-Human-Plasma, Bestell-Nr. ORKL; Dade Behring Marburg GmbH, Deutschland) mit diesem Mangelplasma erstellt wird.

### Figuren:

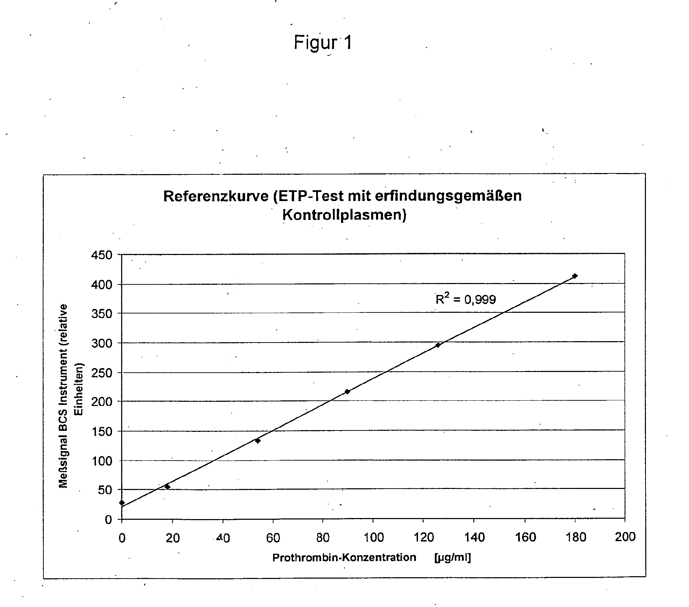

Die Figuren 1 bis 4 dienen zur weiteren Erläuterung der Erfindung.

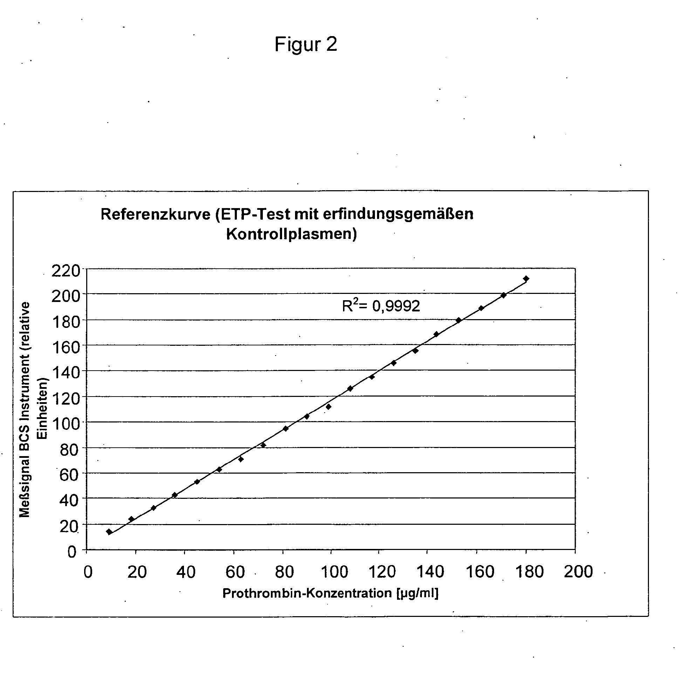

**Fig. 1 und 2** zeigen die Korrelation gemessener ETP-Rohwerte (Messsignal des BCS® Gerätes) mit den Prothrombin-Konzentrationen der gemäß Beispiel 5 hergestellten Kontrollplasmen. Die Prothrombinkonzentration ist als eigentlicher Konzentrationswert des jeweiligen Kontrollplasmas angegeben (µg/ml Prothrombin). Es zeigt sich eine lineare Abhängigkeit der ETP-Messsignale von den Prothrombinkonzentrationen und damit eine einfache Möglichkeit der Kalibrierung, Standardisierung und/oder Kontrolle eines ETP-Tests mittels der erfindungsgemäßen Kontrollplasmen. R²: Korrelationskoeffizient einer zweidimensionalen Zufallsgröße. Mit Hilfe des Korrelationskoeffizienten läßt sich feststellen, ob es eine Beziehung zwischen zwei Eigenschaften gibt.

Bei Fig. 1 wurden 6 lyophilisiert gelagerte Kontrollplasmen und bei Fig. 2 wurden 20 eingefroren gelagerte Kontrollplasmen mit steigender FII-Konzentration verwendet.

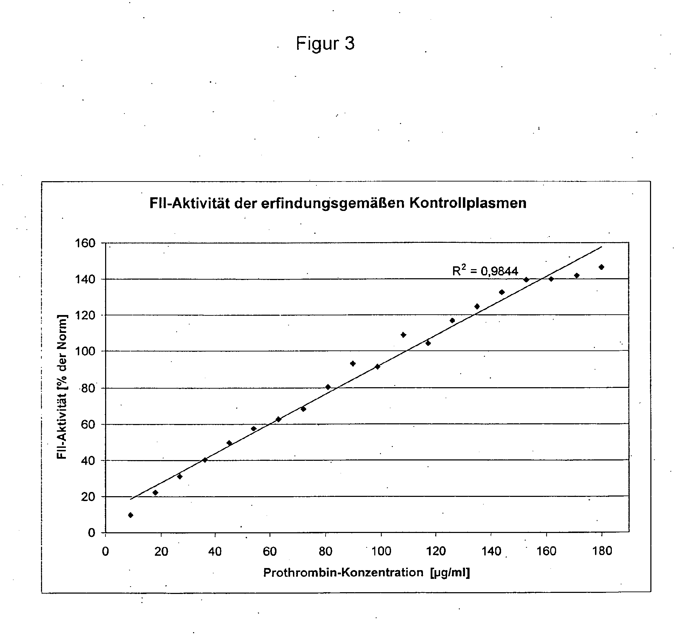

**Fig. 3** zeigt eine zu Fig. 1 analoge Darstellung der FII₋Aktivität (in % der Norm) in Abhängigkeit von der FII-Konzentration (µg/ml) der erfindungsgemäßen Kontrollplasmen. Es ergibt sich eine lineare Abhängigkeit und damit eine sehr einfache Möglichkeit der Kalibration, Standardisierung und/oder Kontrolle von FII- bzw. Flla-Tests mittels der erfindungsgemäßen Kontrollplasmen. Da hier ein FII-Aktivitätstest verwendet wurde, der indirekt über die Gerinnungszeit durch Bildung eines Fibringerinnsel funktioniert, ist die lineare Regression deutlich schlechter als beim ETP Test. Der FII-Aktivitätstest ist nur in einem Konzentrationsbereich von etwa 30 - 140 µg/ml FII direkt proportional zur FII-Konzentration. Der Test wurde gemäß Herstellerangaben unter Verwendung von Innovin® (Dade Behring Marburg GmbH, Deutschland) am BCS®-Gerät (Dade Behring Marburg GmbH, Deutschland) durchgeführt.

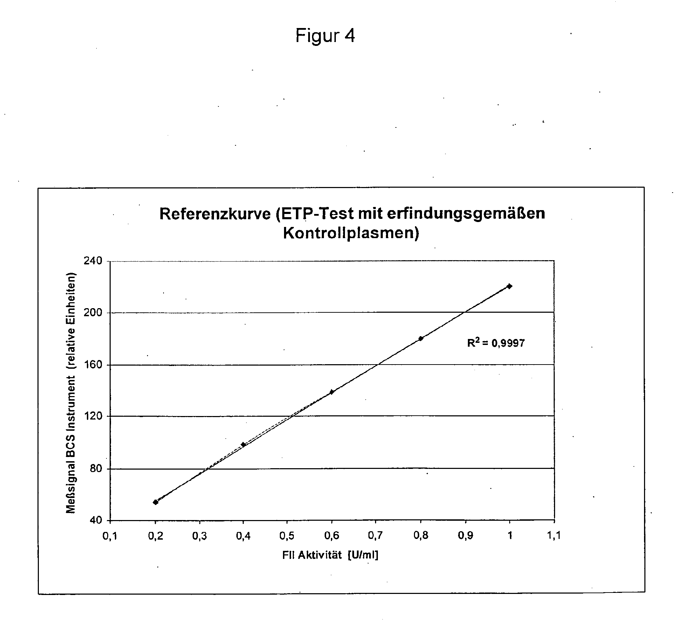

**Fig. 4** zeigt die Korrelation gemessener ETP-Rohwerte (Messsignal des BCS® Gerätes) mit FII-Aktivität der gemäß Beispiel 6 hergestellten Kontrollplasmen. Die Aktivität ist als U/ml des jeweiligen Kontrollplasmas angegeben, wobei ein Normalplasmapool mit 1 U/mL definiert ist. Es zeigt sich eine lineare Abhängigkeit der ETP-Messsignale von der FII-Aktivität und damit eine einfache Möglichkeit der Kalibrierung und/oder Kontrolle eines ETP-Tests mittels der erfindungsgemäßen Kontrollplasmen.

## Patentansprüche

1. Kontrollplasma, bevorzugt humanes Kontrollplasma, mit einer gegenüber Normalplasma, bevorzugt gegenüber humanem Normalplasma, definiert veränderten Prothrombinkonzentration.

2. Kontrollplasma nach Anspruch 1, **dadurch gekennzeichnet, dass** seine Prothrombinkonzentration niedriger oder höher ist als die von Normalplasma.

3. Kontrollplasma nach Anspruch 1 oder 2, **dadurch gekennzeichnet, dass** seine Prothrombinkonzentration höher ist als 1,4 µM, bevorzugt 1,5 bis 5 µM, besonders bevorzugt 1,6 bis 4 µM, ganz besonders bevorzugt 1,7 bis 3 µM.

4. Kontrollplasma nach Anspruch 1 oder 2, **dadurch gekennzeichnet, dass** seine Prothrombinkonzentration niedriger ist als 1,4 µM, bevorzugt 0 bis 1,3 µM, besonders bevorzugt 0,1 bis 1,2 µM, ganz besonders bevorzugt 0,2 bis 1,0 µM.

5. Kontrollplasma nach einem oder mehreren der Ansprüche 1 bis 4, **dadurch gekennzeichnet, dass** es ein oder mehrere Antikoagulantien, eine oder mehrere Puffersubstanzen, einen oder mehrere Proteaseinhibitoren, einen oder mehrere Stabilisatoren und/oder andere in der Plasmaaufbereitung gebräuchliche Hilfs- und Zusatzstoffe enthält.

6. Kontrollplasma nach einem oder mehreren der Ansprüche 1 bis 5, **dadurch gekennzeichnet, dass** als Antikoagulanz Natriumcitrat, Heparin oder EDTA verwendet wird.

7. Kontrollplasma nach einem oder mehreren der Ansprüche 1 bis 6, **dadurch gekennzeichnet, dass** als Puffersubstanz Hepes und/oder Tris verwendet wird.

8. Kontrollplasma nach einem oder mehreren der Ansprüche 1 bis 7, **dadurch gekennzeichnet, dass** ein Proteaseninhibitor verwendet wird.

9. Kontrollplasma nach einem oder mehreren der Ansprüche 1 bis 8, **dadurch gekennzeichnet, dass** als Stabilisator ein Zucker und/oder ein Zuckeralkohol, bevorzugt Mannit, verwendet wird.

10. Kontrollplasma nach einem oder mehreren der Ansprüche 1 bis 9, **dadurch gekennzeichnet, dass** es delipidisiert ist.

11. Kontrollplasma nach einem oder mehreren der Ansprüche 1 bis 10, **dadurch gekennzeichnet, dass** es defibriniert ist.

12. Kontrollplasma nach einem oder mehreren der Ansprüche 1 bis 11, **dadurch gekennzeichnet, dass** es lyophilisiert ist.

13. Delipidisiertes humanes oder delipidisiertes tierisches FII-Mangelplasma, mit einer FII-Konzentration kleiner 5 %, bevorzugt kleiner 1 %, der FII-Konzentration des entsprechenden Normalplasmas.

14. FII-Mangelplasma gemäß Anspruch 13, **dadurch gekennzeichnet, dass** es defibriniert ist.

15. Die Verwendung des FII-Mangelplasmas nach Anspruch 13 oder 14, zur Herstellung eines Kontrollplasmas nach einem der Ansprüche 1 bis 12.

16. Verwendung von einem Kontrollplasma nach einem oder mehreren der Ansprüche 1 bis 12 zur Kalibrierung, Standardisierung und/oder Kontrolle von Thrombinaktivitätstests, Thrombingenerierungsteste, Prothrombintests oder von enzymatischen Tests, bei denen die Thrombinaktivität oder - konzentration über die Aktivierung von Prothrombin bestimmt wird.

17. Verwendung nach Anspruch 16 zur Kalibrierung, Standardisierung und/oder Kontrolle eines ETP-Tests.

18. Verfahren zur Herstellung von einem Kontrollplasma oder einem FII-Mangelplasma nach einem oder mehreren der Ansprüche 1 bis 14, welches gegenüber Normalplasma eine niedrigere FII-Konzentration aufweist, umfassend den Schritt der vollständigen oder teilweisen Entfernung des Prothrombins aus einem Plasma durch Immunadsorption.

19. Verfahren nach Anspruch 18, **dadurch gekennzeichnet, dass** zur Immunadsorption ein monoklonaler oder ein polyklonaler Antikörper verwendet wird.

20. Verfahren zur Delipidisierung eines Kontrollplasmas oder FII-Mangelplasmas nach einem oder mehreren der Ansprüche 1 bis 14 umfassend den Schritt der vollständigen oder teilweisen Entfernung der Lipoproteine, bevorzugt durch Adsorption an lipophile Substanzen.

21. Verfahren zur Herstellung eines Kontrollplasmas nach einem oder mehreren der Ansprüche 1 bis 12 umfassend die Schritte
(a) Zugabe definierter Teile eines Normalplasmas zu einem FII-Mangelplasma oder
(b) Zugabe von reinem Prothrombin und/oder Prothrombin-Konzentrat zu FII-Mangelplasma,
wobei die Zugabe auch in der umgekehrten Reihenfolge erfolgen kann.

22. Kalibrierset, bestehend aus zwei oder mehr Kontrollplasmen unterschiedlicher FII-Konzentration gemäß einem der Ansprüche 1-12.

23. Kalibrierset, bestehend aus einem Kontrollplasma gemäß einem der Ansprüche 1 bis 12 mit einer FII-Konzentration zwischen 1,3 und 3,9 µM und einem Verdünnungsmedium, bevorzugt einem FII-Mangelplasma.
